# EUROPÄISCHE PATENTANMELDUNG

(11) **EP 4 837 544 A2**
(43) Veröffentlichungstag der Anmeldung: **11.11.2026**
(21) Anmeldenummer: 26207876.9
(22) Anmeldetag: 07.05.2024
(51) Int. Cl.: A61F 2/74

(54) **SCHALTVENTIL**

(30) Priorität: 12.05.2023 DE 102023112610; 05.10.2023 DE 102023127105
(62) Dokumentnummer(n) der früheren Anmeldung(en) nach Art. 76 EPÜ: 24725136.6 / 4 709 324
(71) Anmelder: Ottobock SE & Co. KGaA, 37115 Duderstadt (DE)
(72) Erfinder: SCHUH, Andreas, 37115 Duderstadt (DE); GROHS, Thorsten, 37115 Duderstadt (DE); PUSCH, Martin, 37115 Duderstadt (DE); KALTENBORN, Sven, 37115 Duderstadt (DE)
(74) Vertreter: Gramm, Lins & Partner Patent- und Rechtsanwälte PartGmbB

(57) **Zusammenfassung**

Die Erfindung betrifft einen Prothesenfuß mit einem Fußteil, einem schwenkbar an dem Fußteil (38) angeordneten Unterschenkelteil (36) und einem Hydrauliksystem, wobei das Hydrauliksystem zwei Hydraulikkammern (40) aufweist, die durch eine Hydraulikleitung fluidtechnisch verbunden sind, wobei sich in der Hydraulikleitung ein Schaltventil befindet, das ein Ventilgehäuse (2) mit einem Zuflussanschluss (6) und einem Abflussanschluss (8), und eine in einem Wellenraum des Ventilgehäuses (2) bewegbare Schaltwelle (4) aufweist, die in eine erste Position, in der der Zuflussanschluss (6) mit dem Abflussanschluss (8) fluidtechnisch verbunden ist, und in eine zweite Position bringbar ist, in der der Zuflussanschluss (6) von dem Abflussanschluss (8) fluidtechnisch getrennt ist, wobei die Einmündung des Zuflussanschlusses und/oder des Abflussanschlusses eine Ausdrehung, Rundung oder Senkung aufweist.

## Beschreibung

Die Erfindung betrifft einen Prothesenfuß mit einem Fußteil, einem schwenkbar an dem Fußteil angeordneten Unterschenkelteil und einem Hydrauliksystem, wobei das Hydrauliksystem zwei Hydraulikkammern aufweist, die durch eine Hydraulikleitung fluidtechnisch verbunden sind, wobei sich in der Hydraulikleitung ein Schaltventil befindet, das ein Ventilgehäuse mit einem Zuflussanschluss und einem Abflussanschluss, und eine in einem Wellenraum des Ventilgehäuses bewegbare Schaltwelle aufweist, die in eine erste Position, in der der Zuflussanschluss mit dem Abflussanschluss fluidtechnisch verbunden ist, und in eine zweite Position bringbar ist, in der der Zuflussanschluss von dem Abflussanschluss fluidtechnisch getrennt ist.

In vielen Prothesenfüßen werden oft hydraulische Systeme verwendet, um die Bewegung des Fußteils relativ zu dem Unterschenkelteil zu dämpfen und/oder Kräfte von einem Aktuator zu übertragen und so Bewegungen der Bauteile relativ zueinander hervorzurufen. In diesen hydraulischen Systemen werden Ventile für unterschiedliche Anforderungen benötigt und verwendet. Das Hydrauliksystem weist zwei Hydraulikkammern auf, von denen eine als Extensionskammer und eine als Flexionskammer bezeichnet werden kann. In einer bevorzugten Ausgestaltung sind beide Hydraulikkammern in einem einzigen Zylinder angeordnet und durch einen verschiebbaren Kolben voneinander getrennt. Der Kolben und der Zylinder sind so aufeinander abgestimmt und in Form und Kontur korrespondierend ausgebildet, dass der Kolben in dem Zylinder verschoben werden kann. Diese Bewegung des Kolbens kann eine geradlinige Bewegung sein. Bevorzugt ist die Bewegung des Kolbens in dem Zylinder eine Schwenkbewegung, die bevorzugt um die Schwenkachse herum ausgeführt wird, um die das Fußteil und das Unterschenkelteil relativ zueinander verschwenkt werden können. Wird das Fußteil relativ zu dem Unterschenkelteil bewegt, wird auch der Kolben im Inneren des Zylinders bewegt. Dadurch wird das Volumen einer Hydraulikkammer vergrößert und das Volumen der anderen Hydraulikkammer reduziert. Es wird daher Hydraulikfluid des Hydrauliksystems aus der sich verkleinernden Hydraulikammer in die sich vergrößernde Hydraulikkammer geleitet. Dabei muss das Hydraulikfluid durch das Schaltventil geleitet werden.

Eine Art von Ventil, die für derartige Hydrauliksysteme benötigt wird, ist ein Schaltventil. Schaltventile werden in einer Hydraulikleitung verwendet, und sind eingerichtet, diese Hydraulikleitung zu öffnen oder zu schließen. Gattungsgemäße Schaltventile verfügen daher über eine bewegbare Schaltwelle, die in eine erste Position und in eine zweite Position relativ zum Ventilgehäuse gebracht werden kann. In der ersten Position ist der Zuflussanschluss des Ventilgehäuses mit dem Abflussanschluss des Ventilgehäuses fluidtechnisch verbunden. Dies bedeutet, dass Hydraulikflüssigkeit durch das Schaltventil hindurch bewegt werden kann. Es tritt durch den Zuflussanschluss in das Ventilgehäuse ein und verlässt das Ventilgehäuse durch den Abflussanschluss. Dies ist vorzugsweise in beide Richtungen möglich, sodass anhand des Schaltventils allein nicht erkennbar ist, welcher der beiden Anschlüsse als Zuflussanschluss und welcher als Abflussanschluss verwendet wird.

In der zweiten Position ist der Zuflussanschluss von dem Abflussanschluss fluidtechnisch getrennt. Dies bedeutet, dass es dem Hydraulikfluid nicht möglich ist, durch das Schaltventil hindurch bewegt zu werden. Auf diese Weise lässt sich die Hydraulikleitung vollständig blockieren, was für viele Anwendungen der Orthopädietechnik von Vorteil und erwünscht ist.

Die erfindungsgemäße Anwendung eines Hydrauliksystems und eines gattungsgemäßen Schaltventils in einer Hydraulikleitung dieses Hydrauliksystems ist ein Prothesenfuß, der über einen Fußteil und einen Knöchelteil verfügt. Beide sind über ein künstliches Gelenk, ein Prothesen-Knöchelgelenk, miteinander verbunden. Wird der Fußteil relativ zum Knöchelteil um die Schwenkachse dieses Gelenkes verschwenkt, kann auf unterschiedliche Absatzhöhen unterschiedlicher Schuhe eingegangen werden. Diese Bewegung zwischen Fußteil und Knöchelteil, die notwendig ist, sofern die Absatzhöhe eingestellt werden soll, ist jedoch nicht immer erwünscht und vorteilhaft. Ist die Absatzhöhe einmal eingestellt, soll eine weitere Verschwenkung unmöglich gemacht werden. Dies ist über ein Schaltventil der hier beschriebenen Art erreichbar. Wird die Schaltwelle in die erste Position relativ zum Ventilgehäuse gebracht, kann der Fußteil relativ zum Knöchelteil verschwenkt werden. Dabei wird in der Regel Hydraulikfluid von einer Hydraulikkammer des Hydrauliksystems in eine andere Hydraulikkammer des Hydrauliksystems gedrückt. Dazu muss das Hydraulikfluid durch die Hydraulikleitung fließen, in der das Schaltventil der hier beschriebenen Art angeordnet ist. Wenn sich die Schaltwelle in der ersten Position befindet, kann das Hydraulikfluid auf diese Weise zwischen den Kammern fließen und die Absatzhöhe des Prothesenfußes eingestellt werden.

Wird jedoch die Schaltwelle in die zweite Position gebracht, wird, wie bereits dargelegt, der Durchfluss durch die Hydraulikleitung unterbunden. Der Zuflussanschluss des Ventilgehäuses ist vom Abflussanschluss des Ventilgehäuses fluidtechnisch getrennt. Da dadurch kein Hydraulikfluid von einer Hydraulikkammer des Hydrauliksystems in die andere Hydraulikkammer des Hydrauliksystems geleitet werden kann, kann auch das Fußteil des Prothesenfußes relativ zum Knöchelteil nicht verschwenkt werden. Ein solches System und ein entsprechendes gattungsgemäßes Schaltventil sind beispielsweise aus der US 10 821 007 B2 bekannt.

In dieser Ausgestaltung ist die Schaltwelle entlang ihrer Längsrichtung verschiebbar angeordnet, wobei die erste Position und die zweite Position durch eine sogenannte "Ball-Lock"-Lösung gehalten werden können. Die Schaltwelle wird in einer Aufnahme gehalten, in der zwei umlaufende Nuten vorhanden sind, in denen jeweils drei nach radial innen federbelastete Kugeln enthalten sind. Wird die Schaltwelle bewegt, werden diese durch eine weitere Kugel nach außen gedrückt, bis die Schaltwelle die jeweilige Position erreicht hat. In dieser jeweiligen Position werden die Kugeln aufgrund der von den Federn ausgeübten Kraft nach radial innen gedrückt, sodass ein bistabiler Zustand erreicht wird. Die Schaltwelle verfügt über ein erstes Dichtelement, insbesondere eine Dichtung, besonders bevorzugt eine Ringdichtung, das so an der Schaltwelle angeordnet ist, dass es in der zweiten Position der Schaltwelle an der Innenwand eines Wellenraums, in dem die Schaltwelle angeordnet ist, anliegt und so ein Fließen von Hydraulikfluid vom Zuflussanschluss zum Abflussanschluss unmöglich macht. Die Schaltwelle verfügt zudem über zwei weitere Dichtungen, vorzugsweise zwei weitere Ringdichtungen, die den Wellenraum, in dem sich Hydraulikfluid befindet und der im Hydrauliksystem herrschende Druck herrscht, nach außen abdichten.

Nachteilig ist, dass ein solches System und ein derartiges Schaltventil anfällig gegen Verschmutzung von außen sind und insbesondere für den Kontakt mit Wasser, ganz besonders mit Salzwasser nicht geeignet sind. Dadurch ist es beispielsweise für den Träger einer orthopädietechnischen Einrichtung, in der ein solches Schaltventil verwendet wird, kaum möglich, mit der Einrichtung zu baden, beispielsweise im Meer zu schwimmen.

Der Erfindung liegt die Aufgabe zugrunde, ein Schaltventil vorzuschlagen, das die Nachteile aus dem Stand der Technik behebt oder zumindest abmildert.

Die Erfindung löst die gestellte Aufgabe durch einen Prothesenfuß gemäß dem Oberbegriff des Anspruchs 1, der sich dadurch auszeichnet, dass an mindestens einem Ende der Schaltwelle ein Betätigungselement angeordnet ist, wobei zwischen dem Betätigungselement und dem Ventilgehäuse eine Dichtung angeordnet ist, die in beiden Positionen der Schaltwelle einen Zwischenraum zwischen dem Betätigungselement und dem Ventilgehäuse abdichtet. Vorzugsweise ist das Betätigungselement so ausgebildet, dass die Schaltwelle von der ersten Position in die zweite Position oder von der zweiten Position in die erste Position gebracht werden kann, indem das Betätigungselement betätigt wird. Vorzugsweise wird das Betätigungselement betätigt, indem eine Kraft aufgebracht wird. Das Betätigungselement ist dann ein Druckknopf oder ein anderes Druckelement, wobei die aufgebrachte Kraft vorzugsweise entlang der Längsrichtung der Schaltwelle wirkt.

Vorzugsweise ist das Betätigungselement so mit der Schaltwelle verbunden, dass das Betätigungselement ebenfalls bewegt wird, wenn die Schaltwelle aus der ersten Position in die zweite Position oder aus der zweiten Position in die erste Position gebracht wird. Das Betätigungselement ist vorzugsweise zumindest teilweise in einer Vertiefung des Ventilgehäuses angeordnet. Besonders bevorzugt ist das Betätigungselement zumindest in einer der beiden Positionen der Schaltwelle vollständig in der Vertiefung des Ventilgehäuses angeordnet. Dies bedeutet, dass es nicht über die Außenkontur des Ventilgehäuses hinausragt. Zwischen dem Ventilgehäuse und der Schaltwelle liegt ein Zwischenraum, der durch eine Dichtung, vorzugsweise eine Ringdichtung, gegen die Umgebung abgedichtet ist.

Vorzugsweise verfügt die Schaltwelle über ein erstes Dichtelement, vorzugsweise eine erste Ringdichtung, die in der zweiten Position der Schaltwelle den Zuflussanschluss vom Abflussanschluss fluidtechnisch trennt. Eine solche Ausgestaltung ist mit einer einfachen Ringdichtung aus dem Stand der Technik bekannt. Wird die Schaltwelle bewegt, bewegt sich auch das erste Dichtelement und gibt so den Strömungsweg vom Zuflussanschluss zum Abflussanschluss frei. Der Wellenraum, in dem sich die Schaltwelle befindet und durch den das Hydraulikfluid strömt, wenn sich die Schaltwelle in der ersten Position befindet, wird durch zwei Begrenzungsdichtungen so abgedichtet, dass Hydraulikfluid diesen Raum nicht verlassen kann. Die Dichtung, die den Zwischenraum zwischen dem Betätigungselement und dem Ventilgehäuse abdichtet, ist zusätzlich zu dem ersten Dichtelement und den Begrenzungsdichtungen vorhanden.

In einer bevorzugten Ausgestaltung ist an beiden Enden der Schaltwelle jeweils ein Betätigungselement angeordnet, wobei zwischen jedem Betätigungselement und dem Ventilgehäuse eine Dichtung angeordnet ist, die in beiden Positionen der Schaltwelle einen Zwischenraum zwischen dem jeweiligen Betätigungselement und dem Ventilgehäuse abdichtet. Besonders bevorzugt handelt es sich bei beiden Betätigungselementen um einen Druckknopf, der so ausgebildet ist, dass ein bezüglich der Längsrichtung der Schaltwelle in axialer Richtung aufgebrachter Druck dazu führt, dass die Schaltwelle aus der ersten Position in die zweite Position oder aus der zweiten Position in die erste Position gebracht wird. Besonders bevorzugt ist dabei jedes Betätigungselement für nur eine Richtung dieser Positionsänderung zu verwenden. Das Betätigungselement an einem ersten Ende der Schaltwelle ist beispielsweise so eingerichtet, dass ein Betätigen dieses Betätigungselementes die Schaltwelle aus der ersten Position in die zweite Position bringt. Das Betätigungselement am gegenüberliegenden zweiten Ende der Schaltwelle ist dann vorzugsweise so eingerichtet, dass ein Betätigen dieses Betätigungselementes die Schaltwelle aus der zweiten Position in die erste Position bringt.

Vorzugsweise ist zwischen dem Ventilgehäuse und jeweils einem der Betätigungselemente ein Volumen eingeschlossen, sodass bevorzugt zwei Volumina vorhanden sind, von denen jedes an einem Ende der Schaltwelle liegt und zwischen dem Ventilgehäuse und einem der beiden Betätigungselemente eingeschlossen ist. Das Volumen wird von der jeweiligen Dichtung, die den Zwischenraum zwischen dem jeweiligen Betätigungselement und dem Ventilgehäuse abgedichtet, verschlossen und abgedichtet. In einer bevorzugten Ausgestaltung sind die beiden Volumina durch einen Kanal fluidtechnisch miteinander verbunden, der vorzugsweise innerhalb der Schaltwelle oder in dem Ventilgehäuse vorhanden ist und vorzugsweise sich über die gesamte Länge der Schaltwelle erstreckt.

Bevorzugt ist die Schaltwelle von der ersten Position in die zweite Position oder von der zweiten Position in die erste Position bringbar, indem sie entlang ihrer Längsrichtung verschoben wird. Dies hat insbesondere für den Fall, dass die Verschiebung der Schaltwelle auch die Verschiebung der Betätigungselemente bedeutet, zur Folge, dass eines der beiden Volumina reduziert und das andere vergrößert wird. Da beide Volumina gegen die Umgebung abgedichtet sind, hätte dies eine Druckveränderung zur Folge, durch die eine Kraft hervorgerufen würde, die der Bewegung der Schaltwelle, die zu dieser Veränderung der beiden Volumina führt, entgegen gerichtet ist. Durch den im Innern der Schaltwelle vorhandenen Kanal, der die beiden Volumina miteinander verbindet, kann dieser Effekt vermieden werden.

Wird ein Betätigungselement betätigt, in dem ein Druck ausgeübt wird, und die Schaltwelle entlang ihrer Längsrichtung verschoben, wird das Volumen zwischen dem betätigten Betätigungselement und dem Ventilgehäuse verkleinert. Da gleichzeitig auch das andere Betätigungselement, das am gegenüberliegenden Ende der Schaltwelle angeordnet ist, bewegt wird, wird das Volumen zwischen dem anderen Betätigungselement und dem Ventilgehäuse vergrößert. Vorzugsweise sind die Volumina so ausgebildet, dass das Gesamtvolumen, das aus den beiden Volumina und dem Volumen des Kanals gebildet wird, unabhängig von der Position der Schaltwelle konstant bleibt. So kann der Druck innerhalb dieses Gesamtvolumens ebenfalls konstant gehalten werden und eine durch Druckdifferenzen hervorgerufene Kraft kann nicht entstehen.

In einer vorteilhaften Ausgestaltung ist die Schaltwelle von der ersten Position in die zweite Position oder umgekehrt bringbar, indem sie um ihre Längsrichtung gedreht wird. Vorzugsweise ist die erste Dichtung, die in der zweiten Position der Schaltwelle den Zuflussanschluss vom Abflussanschluss trennt, in diesem Fall eine schräg angeordnete Ringdichtung, beispielsweise ein O-Ring. Vorzugsweise verfügt die Schaltwelle über eine umlaufende Nut, in der die Ringdichtung angeordnet ist. Während eine solche Nut im Stand der Technik einen Kreis in einer Ebene bildet, die senkrecht auf der Längserstreckung der Schaltwelle liegt, ist diese Ebene bei der hier beschriebenen Ausführungsform vorzugsweise gekippt, besonders bevorzugt um 45° gekippt. Wird die Schaltwelle in dieser Position gedreht, beispielsweise um 180° um ihre Längsrichtung gedreht, verändert sich ihre Lage und Orientierung relativ zum Ventilgehäuse. Die Position, an der der Zuflussanschluss und der Abflussanschluss in den Wellenraum münden, die auch Einmündung genannt wird, ist dabei so gewählt, dass sie in der zweiten Position der Schaltwelle und damit auch der ersten Dichtung auf unterschiedlichen Seiten der ersten Dichtung, vorzugsweise des O-Rings, liegen und in der ersten Position der Schaltwelle auf der gleichen Seite der ersten Dichtung.

Vorzugsweise weist das erste Dichtelement einen Dichtring auf, der so auf der Schaltwelle angeordnet ist, dass die durch den Dichtring definierte Ebene einen Winkel von wenigstens 20°, vorzugsweise wenigstens 30°, besonders bevorzugt wenigstens 40° und höchstens 70°, bevorzugt höchstens 60°, besonders bevorzugt 50° einschließt. Der Dichtring liegt vorzugsweise vollständig in einer Ebene, die daher durch diesen Dichtring definiert wird. In der Regel werden Dichtringe so angeordnet, dass sich die Schaltwelle orthogonal zu der durch den Dichtring definierten Ebene erstreckt. Dies ist bei diesem Ausführungsbeispiel anders. Durch die Drehung der Schaltwelle in dem Wellenraum relativ zu dem Ventilgehäuse verändert sich die Stelle der Wandung des Wellenraumes, die mit dem Dichtring in Kontakt kommt. In der ersten Position der Schaltwelle verläuft diese Kontaktstelle entlang einer ersten Linie. In der zweiten Position der Schaltwelle, die in diesem Ausführungsbeispiel dadurch erreicht wird, dass die Schaltwelle um ihre Längsachse gedreht wird, verläuft diese Kontaktstelle entlang einer zweiten Linie, die von der ersten Linie verschieden ist. Dabei liegen die erste Linie und die zweite Linie auf unterschiedlichen Seiten des Zuflussanschlusses oder des Abflussanschlusses.

Vorzugsweise ist das Betätigungselement in diesem Fall ein Drehknopf, der beispielsweise manuell betätigt werden kann, indem er gedreht wird. Vorzugsweise ist der Drehknopf drehfest mit der Schaltwelle verbunden, sodass eine Drehung des so ausgebildeten Betätigungselementes auch eine Drehung der Schaltwelle zur Folge hat, die dadurch aus der ersten Position in die zweite Position oder umgekehrt gebracht wird. Alternativ dazu ist das Betätigungselement als Druckknopf ausgebildet, der betätigt wird, in dem eine in Längsrichtung der Schaltwelle wirkende Kraft ausgeübt wird. Vorzugsweise weist die Schaltwelle an dem Ende, an dem ein solches Betätigungselement angeordnet ist, ein Gewinde auf. Das Betätigungselement verfügt über ein korrespondierendes Gegengewinde. Vorzugsweise ist das Gewinde als Außengewinde auf der Schaltwelle ausgebildet. Entsprechend ist das Gegengewinde vorzugsweise ein Innengewinde. Das Gewinde und das Gegengewinde sind dabei so ausgebildet, dass keine Selbsthemmung auftritt. Eine Bewegung des Betätigungselementes in axialer Richtung der Schaltwelle, die beispielsweise durch ausgeübten Druck hervorgerufen wird, wird dann durch die beiden Gewinde in eine Rotationsbewegung der Schaltwelle umgesetzt, wodurch diese von der ersten Position in die zweite Position oder umgekehrt gebracht wird.

In einer besonders bevorzugten Ausgestaltung verfügt die Schaltwelle an beiden Enden über jeweils ein Betätigungselement, das als solches Druckelement, beispielsweise Druckknopf, ausgebildet ist. Die Schaltwelle verfügt dann vorzugsweise an beiden Enden über ein Gewinde und beide Betätigungselemente verfügen über ein entsprechendes Gegengewinde. Wird nun eines der Betätigungselemente in axialer Richtung verschoben, vorzugsweise in das Ventilgehäuses hineingedrückt, wird diese longitudinale Bewegung in eine Rotationsbewegung der Schaltwelle umgesetzt. Diese hat vorzugsweise zur Folge, dass das am gegenüberliegenden Ende angeordnete andere Betätigungselement ebenfalls longitudinal bewegt und besonders bevorzugt aus der Vertiefung des Ventilgehäuses hinaus bewegt wird. Auch in dieser Ausgestaltung kann folglich erreicht werden, dass zwischen jeweils einem der Betätigungselemente und dem Ventilgehäuse ein bereits beschriebenes Volumen vorhanden ist, wobei eines dieser Volumina verkleinert und das andere der Volumina vergrößert wird, wenn die Schaltwelle aus der ersten Position in die zweite Position oder umgekehrt gebracht wird.

Alternativ dazu sind die beiden Betätigungselemente derart angeordnet, dass sie sich aufeinander zu bewegen, wenn der Druck auf eines der Betätigungselemente ausgeübt wird. Dies bedeutet, dass sich beide Betätigungselemente relativ zu einem anderen Element des Schaltventils, beispielsweise der Schaltwelle oder dem Ventilgehäuse bewegen. Dies lässt sich beispielsweise erreichen, indem die Schaltwelle an beiden Enden über ein Gewinde verfügt, die gegenläufig zueinander ausgebildet sind. Auch die beiden Betätigungselemente verfügen jeweils über ein Gewinde, wobei auch diese beiden Gewinde gegenläufig sind.

Vorteilhafterweise ist an wenigstens einem der Betätigungselemente und/oder an dem Ventilgehäuse wenigstens ein Permanentmagnet angeordnet, der mit einem entsprechenden Gegenelement eine Kraft erzeugt, die die Schaltwelle in die erste Position oder die zweite Position vorspannt. Das Gegenelement ist beispielsweise als magnetisierbares Element, beispielsweise aus einem magnetisierbaren Metall ausgebildet. Wird der Permanentmagnet in die Nähe des Gegenelementes gebracht, entsteht durch die magnetische Wechselwirkung eine anziehende Kraft. Alternativ oder zusätzlich dazu ist das Gegenelement ebenfalls als Permanentmagnet ausgebildet. Dieser ist dabei so angeordnet, dass ungleichnamige Pole aufeinander zu gerichtet sind, sodass eine anziehende Wechselwirkung zwischen den beiden Permanentmagneten hervorgerufen wird. Vorzugsweise sind mehrere solcher Paare aus Permanentmagnet und Gegenelement vorhanden.

Insbesondere für den Fall, dass eines oder beide der Betätigungselemente beim Betätigen in eine Vertiefung im Ventilgehäuse hinein bewegt werden oder auf andere Weise auf das Ventilgehäuse zu bewegt werden, ist es sinnvoll, eine magnetische Wechselwirkung hervorzurufen und entsprechende Bauteile, wie oben beschrieben, anzuordnen. Die magnetische anziehende Wechselwirkung wird in diesem Fall immer dann am größten, wenn das jeweilige Betätigungselement betätigt wurde und die Schaltwelle ihre neue Position erreicht hat. Dies hat zur Folge, dass die Wechselwirkung und die dabei hervorgerufene Kraft die Schaltwelle in ihrer neuen Position halten, sodass ein versehentliches Entfernen der Schaltwelle aus dieser Position erschwert oder vollständig verhindert wird.

Bevorzugt ist die Einmündung des Zuflussanschlusses in den Wellenraum und/oder die Einmündung des Abflussanschlusses in den Wellenraum mit einer umlaufenden Ausdrehung verrundet. Dies bedeutet, dass sich der Durchmesser des jeweiligen Anschlusses im Bereich der Einmündung vergrößert. Zudem ist am Übergang zwischen der Wandung des Wellenraumes und der Wandung des jeweiligen Anschlusses keine scharfe Kante, sondern eine Rundung vorhanden. Wird die Schaltwelle von der ersten Position in die zweite Position oder umgekehrt gebracht, muss das erste Dichtelement mit der Schaltwelle bewegt werden. Es wird dabei über den Zuflussanschluss oder den Abflussanschluss hinwegbewegt. Durch die Verrundung der Einmündung wird das erste Dichtelement bei dieser Bewegung deutlich weniger mechanisch beansprucht und daher geschont. Diese Rundung, die auch als Senkung bezeichnet werden kann, ist vorzugsweise eine kugelförmige Senkung. Der als erstes Dichtelement vorzugsweise verwendete Dichtring, insbesondere O-Ring, gleitet dann besser über diese Einmündungen und es kommt zu weniger Verschleiß an der Dichtung. Außerdem werden eine leichtere Bewegbarkeit und eine geringere Gefahr der Verklemmung beim Bewegen der Schaltwelle aus einer Position in die jeweils andere Position erreicht.

Diese Ausgestaltung ist unabhängig davon von Vorteil, ob ein Zwischenraum zwischen dem Betätigungselement und dem Ventilgehäuse durch eine Dichtung abgedichtet ist, oder wie eine solche Dichtung ausgebildet ist.

Ein Prothesenfuß mit einem Fußteil, einem schwenkbar an dem Fußteil angeordneten Unterschenkelteil und einem Hydrauliksystem, wobei das Hydrauliksystem zwei Hydraulikkammern aufweist, die durch eine Hydraulikleitung fluidtechnisch verbunden sind, wobei sich in der Hydraulikleitung ein Schaltventil befindet, das ein Ventilgehäuse mit einem Zuflussanschluss und einem Abflussanschluss, und eine in einem Wellenraum des Ventilgehäuses bewegbare Schaltwelle aufweist, die in eine erste Position, in der der Zuflussanschluss mit dem Abflussanschluss fluidtechnisch verbunden ist, und in eine zweite Position bringbar ist, in der der Zuflussanschluss von dem Abflussanschluss fluidtechnisch getrennt ist, stellt daher eine eigene Erfindung dar, wenn die Einmündung des Zuflussanschlusses und/oder des Abflussanschlusses eine hier beschriebene Ausdrehung, Rundung oder Senkung aufweist.

Die in dieser Anmeldung beschriebenen Merkmale können einzeln, in jeder denkbaren Kombination oder insgesamt mit dieser eigenen Erfindung kombiniert werden.

Vorzugsweise wird das Betätigungselement betätigt, indem es verschoben wird, wobei es vorzugsweise parallel zur Längsrichtung der Schaltwelle verschoben wird. Es ist dabei von Vorteil, wenn das jeweilige Betätigungselement verdrehsicher in einer Aufnahme des Ventilgehäuses geführt ist. Dies hat zur Folge, dass das Betätigungselement nicht oder nur in vorbestimmter Form zusätzlich zu der Längsverschiebung, die zur Betätigung des Betätigungselementes nötig ist, gedreht werden kann. Dies ist besonders einfach dadurch erreichbar, dass das Betätigungselement und die Aufnahme, in der es bewegt werden kann, einen zueinander korrespondierend ausgebildeten Querschnitt aufweisen, der nicht kreisförmig ist. Vorzugsweise ist er oval und/oder weist zumindest einen Führungsvorsprung, beispielsweise einen Führungsstift auf. Dieser Vorsprung oder Stift ist in einer entsprechenden Vertiefung oder Nut in der Wandung der Aufnahme angeordnet und verhindert ein Verdrehen des Betätigungselementes relativ zu der Aufnahme.

Vorzugsweise verfügt das Schaltventil, besonders bevorzugt die Schaltwelle, über einen Rücksteller, der eingerichtet ist, die Schaltwelle aus der ersten Position in die zweite Position zu bringen, wenn sich die Schaltwelle eine vorbestimmte Dauer in der ersten Position befunden hat. In der zweiten Position ist ein Durchfluss von Hydraulikfluid durch das Schaltventil nicht oder nur sehr eingeschränkt möglich. Dies bedeutet in den meisten orthopädietechnischen Einrichtungen, dass eine Bewegung zwischen zwei miteinander verbundenen Bauelementen nicht möglich ist. Dies sorgt für eine erhöhte Stabilität und wird daher in der Regel als Standardzustand (default) bezeichnet. Es ist von Vorteil, wenn dieser Zustand automatisch erreicht und eingenommen werden kann. Daher wird in die Schaltwelle aus der ersten Position, in der Hydraulikfluid das Ventil passieren kann, in die zweite Position gebracht, nachdem die Schaltwelle für die vorbestimmte Dauer in der ersten Position gewesen ist. Dies wird auch als Zeitverzögerung oder Zeitverzögerungsglied bezeichnet.

Dabei handelt es sich vorzugsweise um eine hydraulische Zeitverzögerung, die besonders bevorzugt einstellbar ist. Die Einstellbarkeit bezieht sich dabei auf die Länge der zeitlichen Dauer, nach der die Schaltwelle in die zweite Position gebracht wird. Alternativ oder zusätzlich dazu verfügt der Rücksteller über einen mechatronischen Antrieb, der die Schaltwelle in die erste und/oder die zweite Position bringt.

In einer bevorzugten Ausgestaltung weist die Schaltwelle einen Abschnitt mit verringertem Querschnitt, beispielsweise eine Abflachung, die beispielsweise einseitig oder zweiseitig ausgebildet ist, oder eine Ausnehmung, Nut und/oder eine symmetrische oder asymmetrische Ausdrehung zur Mittelachse der Schaltwelle auf, durch den Hydraulikfluid von dem Zuflussanschluss zu dem Abflussanschluss strömen kann, wenn sich die Schaltwelle in der ersten Position befindet.

Vorzugsweise ist die Schaltwelle in der ersten Position und/oder der zweiten Position federbelastet. Eine dafür verwendete Feder wirkt vorzugsweise unterstützend für das Schalten der Schaltwelle in die sperrende zweite Position und/oder zum Halten der ersten und/oder der zweiten Position dienen. Die Feder ist vorzugsweise in einem Volumen zwischen einem Betätigungselement und dem Ventilgehäuse angeordnet. Vorzugsweise ist zwischen dem Ventilgehäuse und jedem vorhandenen Betätigungselement ein Federelement angeordnet.

Mithilfe der beigefügten Figuren werden nachfolgend einige Ausführungsbeispiele der vorliegenden Erfindung näher erläutert. Es zeigen:
- Figuren 1 und 2: - ein Schaltventil gemäß einem ersten Ausführungsbeispiel der vorliegenden Erfindung mit der Schaltwelle in unterschiedlichen Positionen
- Figuren 3 und 4: - die Schaltwelle aus den Figuren 1 und 2 in einer 3-dimensionalen und einer Schnitt-Darstellung,
- Figuren 5 und 6: - ein Schaltventil gemäß einem weiteren Ausführungsbeispiel mit der Schaltwelle in unterschiedlichen Positionen,
- Figuren 7 und 8: - ein Schaltventil gemäß einem weiteren Ausführungsbeispiel mit der Schaltwelle in unterschiedlichen Positionen
- Figuren 9 und 10: - einen Prothesenfuß, in dem ein Schaltventil verbaut ist, in einer Seitenansicht und einer Schnittdarstellung;
- Figur 11: - die Schnittsdarstellung durch ein Schaltventil gemäß einem weiteren Ausführungsbeispiel der vorliegenden Erfindung und
- Figuren 12 und 13: - den Ausschnitt einer Schnittdarstellung durch ein Schaltventil gemäß einem weiteren Ausführungsbeispiel der vorliegenden Erfindung.

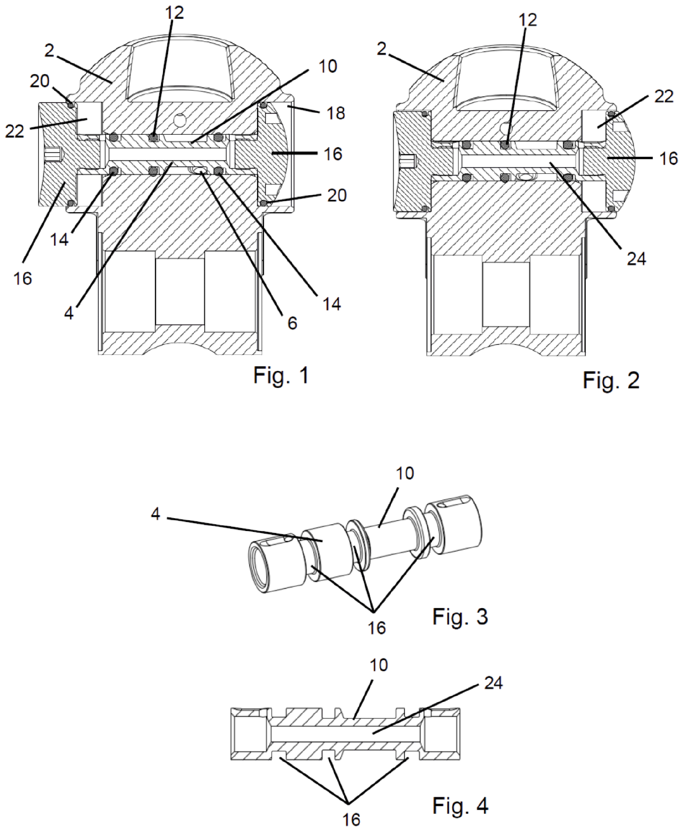

Figur 1 zeigt ein Schaltventil gemäß einem ersten Ausführungsbeispiel der vorliegenden Erfindung. Es verfügt über ein Ventilgehäuse 2, in dem sich eine Schaltwelle 4 befindet. Die Schaltwelle 4 ist im gezeigten Ausführungsbeispiel nach links und rechts in einem Wellenraum verschiebbar. Figur 1 zeigt die Schaltwelle in der ersten Position. Ein Zuflussanschluss 6 mündet in den Wellenraum. Ein in Figur 1 nicht dargestellter Abflussanschluss 8 mündet ebenfalls in den Wellenraum, die zugehörige Einmündung ist in der in den Figuren 1 und 2 dargestellten Ansicht nicht zu sehen. Die Schaltwelle 4 weist in den Figuren 1 und 2 eine Verjüngung 10, also einen Abschnitt mit verringertem Querschnitt auf. In der in Figur 1 gezeigten Situation, in der sich die Schaltwelle 4 in der ersten Position befindet, kann Hydraulikfluid, das aus dem Zuflussanschluss 6 in den Wellenraum eindringt, in dem Bereich der Verjüngung 10 zwischen der Schaltwelle 4 und der Wandung des Wellenraumes zum Abflussanschluss fließen.

Die Schaltwelle weist ein erstes Dichtelement 12 in Form eines um die Schaltwelle 4 umlaufenden Dichtringes vorzugsweise eines O-Ringes auf. Sowohl die Einmündung des Zuflussanschlusses 6 als auch die des Abflussanschlusses 8 befinden sich auf der gleichen Seite des ersten Dichtelementes 12, im gezeigten Ausführungsbeispiel links davon. Ein Fluidfluss von dem Zuflussanschluss 6 zum Abflussanschluss 8 wird daher durch das erste Dichtelement 12 in der Situation in Figur 1 nicht unterbunden. Der Wellenraum, in dem sich Hydraulikfluid befinden kann, wird auf beiden Seiten durch eine Begrenzungsdichtung 14 begrenzt und abgedichtet.

An den Enden der Schaltwelle 4 ist jeweils ein Betätigungselement 16 angeordnet, das in einer dafür vorgesehenen Ausnehmung 18 des Ventilgehäuses 2 geführt ist. Man erkennt in Figur 1, dass die Schaltwelle nach links in die erste Position verschoben wurde, so dass das linke Betätigungselement 16 aus seiner Ausnehmung 18 heraussteht. Das rechte Betätigungselement 16 hingegen ist in seine Ausnehmung 18 hineinbewegt worden. Der Zwischenraum zwischen dem Betätigungselement 16 und dem Ventilgehäuse 2 ist jeweils durch eine Dichtung 20 abgedichtet.

Wird in der in Figur 1 gezeigten Situation ein entlang der Längsrichtung der Schaltwelle 4 gerichteter Druck, im gezeigten Ausführungsbeispiel nach rechts, auf das linke Betätigungselement 16 ausgeübt, wird die Schaltwelle 4 aus der in Figur gezeigten ersten Position in die zweite Position gebracht, die in Figur 2 dargestellt ist.

Man erkennt, dass in Figur 2 das linke Betätigungselement 16 in seine Ausnehmung 18 hineinbewegt wurde und dass das rechte Betätigungselement 16 über den Rand seiner Ausnehmung 18 hinaussteht. Durch die Verschiebung der Schaltwelle 4 wurde auch das erste Dichtelement 12 verschoben. Es befindet sich nun zwischen der Einmündung des Zuflussanschlusses 6 und der Einmündung des Abflussanschlusses 8 und blockiert so einen Fluss von Hydraulikfluid durch den Wellenraum.

In Figur 1 befindet sich zwischen dem linken Betätigungselement 16 und dem Ventilgehäuse 2 ein Volumen 22. Dieses Volumen 22 wird verkleinert, wenn die Schaltwelle 4 in die zweite Position verschoben wird. In Figur 2 ist zu erkennen, dass in der zweiten Position der Schaltwelle 4 ein Volumen 22 zwischen dem rechten Betätigungselement 16 und dem Ventilgehäuse 2 befindet. Durch geschickte Wahl der Abmessungen der Betätigungselemente 16 und der Ausnehmungen 18 ist die kombinierte Gesamtgröße der beiden Volumina 22 konstant. Um einen Ausgleich eines Fluids, beispielsweise Luft, zwischen den beiden Volumina 22 zu erlauben, weist die Schaltwelle 4 einen Kanal 24 auf, durch den die beiden Volumina 22 miteinander verbunden sind.

Figur 3 zeigt die Schaltwelle 4 in einer 3-dimensionalen Ansicht. Man erkennt die Verjüngung 10. Man erkennt zudem drei Nuten 26. In der mittleren Nut 26 wird das erste Dichtelement 12 und in den beiden anderen Nuten 26 jeweils eine Begrenzungsdichtung 14 angeordnet. Figur 4 zeigt die Schaltwelle 4 in einer Schnittdarstellung, in der zusätzlich der Kanal 24 zu erkennen ist.

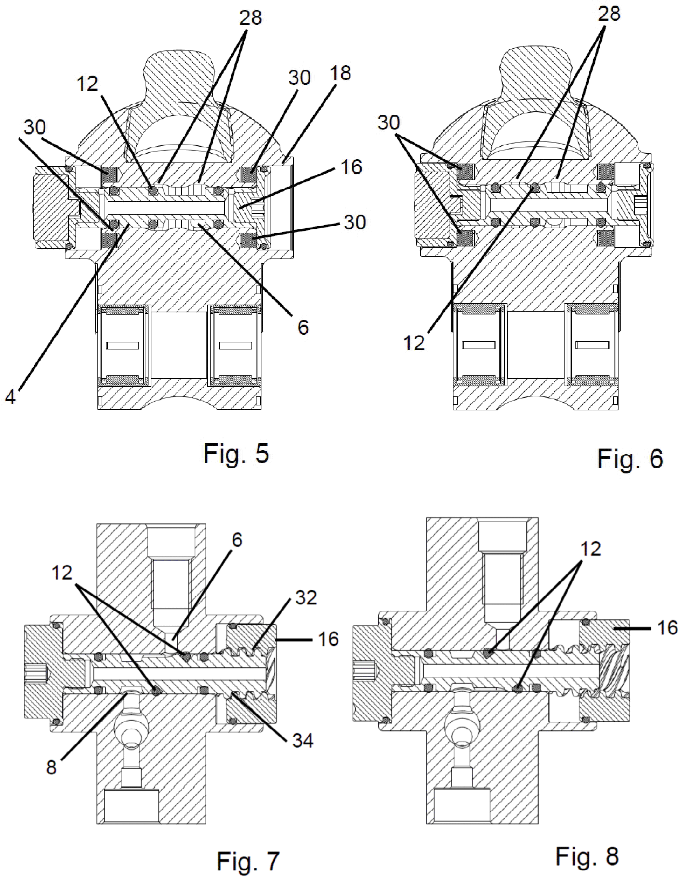

In den Figuren 5 und 6 ist ein weiteres Ausführungsbeispiel der vorliegenden Erfindung dargestellt. Es entspricht in seiner Funktion dem Schaltventil aus den Figuren 1 und 2. Es wird daher nur auf Unterschiede eingegangen.

Die Schaltwelle 4 ist in einem Wellenraum angeordnet, der über zwei Ausbuchtungen 28 verfügt. In Figur 5 ist die Schaltwelle 4 in der ersten Position dargestellt. Die Einmündung des Zuflussanschlusses 6 und die nicht dargestellte Einmündung des Abflussanschlusses liegen auf der gleichen Seite (im gezeigten Ausführungsbeispiel rechts) des ersten Dichtelementes 12. Diese ist im Bereich einer der beiden Ausbuchtungen 28 und hat im gezeigten Ausführungsbeispiel keinen Kontakt zur Wandung des Wellenraumes. Es ist daher möglich, dass Hydraulikfluid von dem Zuflussanschluss zu dem Abflussanschluss strömt. In Figur 5 ist das rechte Betätigungselement 16 in seiner Aufnahme 18 angeordnet. Am Boden der beiden Ausnehmungen 18 sind jeweils zwei Magnete 30 positioniert. Die Betätigungselemente 16 sind aus einem magnetischen oder magnetisierbaren Material hergestellt oder beinhalten ein solches Material, so dass eine magnetische Anziehung zwischen den Magneten 30 und den Betätigungselementen die jeweilige Position der Schaltwelle 4 stabilisiert.

In Figur 6 ist die Schaltwelle 4 in ihrer zweiten Position dargestellt. Das erste Dichtelement 12 ist nun zwischen der Einmündung des Zuflussanschlusses 6 und der Einmündung des Abflussanschlusses angeordnet und unterbindet so einen Fluidfluss durch den Wellenraum. Das erste Dichtelement 12 kommt in dieser Position der Schaltwelle 4 mit der Wandung des Wellenraums zwischen den beiden Ausbuchtungen 28 in Kontakt. In Figur 6 üben die linken Magnete 30 eine Anziehungskraft auf das linke Betätigungselement 16 auf und halten es und damit die Schaltwelle 4 in der gezeigten zweiten Position.

In den Figuren 7 und 8 ist ein Schaltventil gemäß einem weiteren Ausführungsbeispiel der vorliegenden Erfindung dargestellt. Die Schaltwelle 4 ist in Figur 7 in der ersten Position und in Figur 8 in der zweiten Position dargestellt. Das erste Dichtelement 12 ist wie in den vorhergehenden Ausführungsbeispielen als O-Ring ausgebildet, der allerdings schräg oder verkippt an der Schaltwelle angeordnet ist. In Figur 7 befindet sich die Einmündung des Zuflussanschlusses 6 und die Einmündung des Abflussanschlusses 8 auf derselben Seite (im gezeigten Ausführungsbeispiel links) des ersten Dichtelementes 12, so dass dieses einen Fluidfluss durch das Schaltventil nicht unterbinden kann. Das Betätigungselement 16 ist mit einem Innengewinde 32 ausgestattet. Die Schaltwelle 4 verfügt über ein korrespondierendes Außengewinde 34. Die Gewinde 32, 34 sind nicht selbsthemmend ausgebildet.

In Figur 8 ist die Schaltwelle 4 in ihrer zweiten Position dargestellt. Das erste Dichtelement 12 ist mit der Schaltwelle 4 gedreht worden und verläuft nun zwischen der Einmündung des Zuflussanschlusses 6 und der Einmündung des Abflussanschlusses 8 und blockiert somit den Fluidfluss durch das Ventil. Wird in der in Figur 8 gezeigten zweiten Position der Schaltwelle 4 ein Druck auf das Betätigungselement 16 ausgeübt, wird das Betätigungselement 16 im gezeigten Ausführungsbeispiel nach links verschoben. Durch die in Eingriff stehenden Gewinde 32, 34 führt diese Verschiebung des Betätigungselementes 16 zu einer Rotation der Schaltwelle 4 um ihre Längsachse, wodurch sie aus der gezeigten zweiten Position in die erste Position gebracht wird.

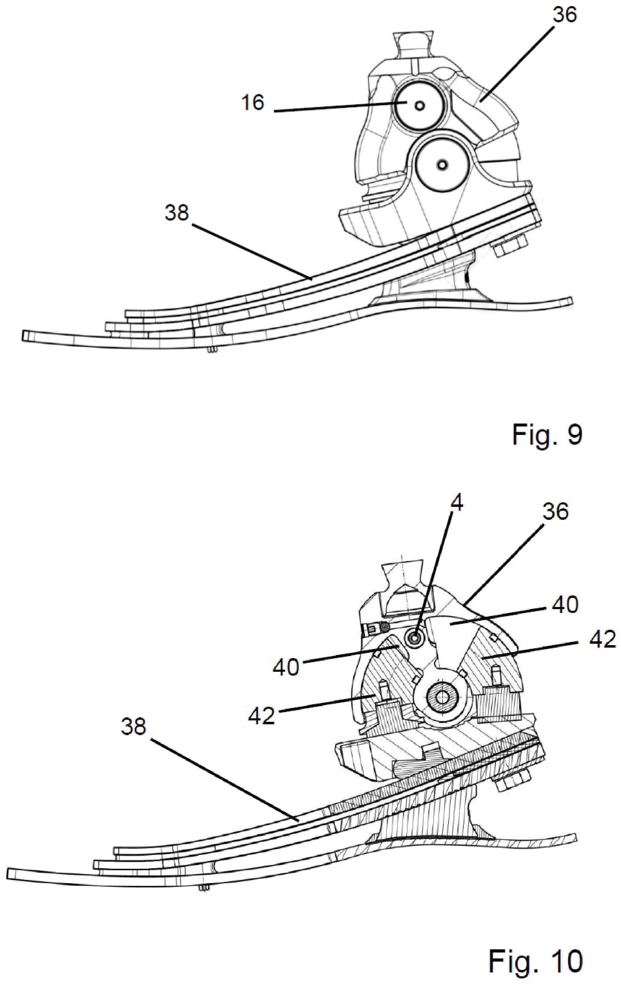

Figur 9 zeigt einen Prothesenfuß mit einem Unterschenkelteil 36 und einem Fußteil 38. Beide sind schwenkbar aneinander angeordnet, wobei beim Verschwenken der beiden Bauteile relativ zueinander Hydraulikfluid durch ein Schaltventil gemäß einem Ausführungsbeispiel der vorliegenden Erfindung bewegt werden muss, das in dem Prothesenfuß verbaut ist. Man erkennt eines der Betätigungselemente 16.

In Figur 10 ist der Prothesenfuß in einer Schnittdarstellung gezeigt. Man erkennt das Unterschenkelteil 36 und das Fußteil 38. Zudem sind die beiden Hydraulikkammern 40 dargestellt, die im gezeigten Ausführungsbeispiel durch Begrenzungselemente 42 begrenzt werden. In der zwischen den beiden Hydraulikkammern 40 liegenden Wand, die als Kolben dient, ist die Schaltwelle 4 zu erkennen, die sich in der gezeigten Darstellung senkrecht zur Zeichenebene erstreckt.

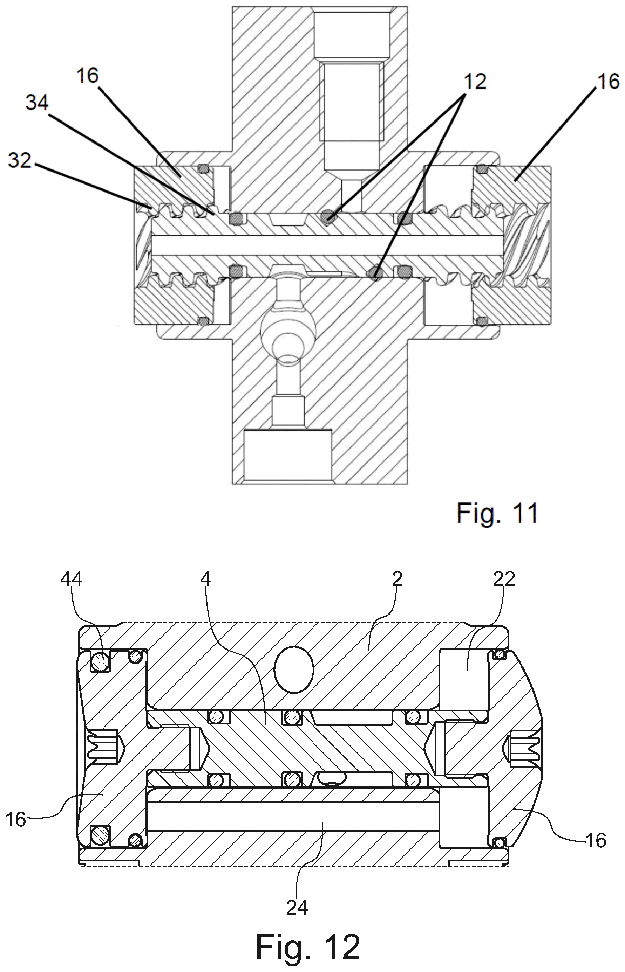

Figur 11 ähnelt der Darstellung aus Figur 8, wobei in Figur 11 an beiden Enden der Schalwelle jeweils ein Betätigungselement 16 angeordnet ist. Die Schaltwelle 4 weist an beiden Enden ein außen an der Schaltwelle 4 angeordnetes Außengewinde 34 und die beiden Betätigungselemente weisen ein entsprechend gestaltetes innen angeordnetes Innengewinde 32 auf. Sind die beiden Außengewinde 34 gegenläufig ausgebildet, hat ein Druck auf eines der beiden Betätigungselemente 16 zur Folge, dass beide Betätigungselemente 16 nach innen, also aufeinander zu, verschoben werden. Anderenfalls wird das jeweils nicht mit Druck beaufschlagte Betätigungselement 16 nach außen bewegt. In dieser bevorzugten Ausgestaltung kann die Luft aus der rechten Kammer 22 in die linke Kammer 22 strömen und umgekehrt. Eine Verbindung zur Atmosphäre ist daher nicht erforderlich.

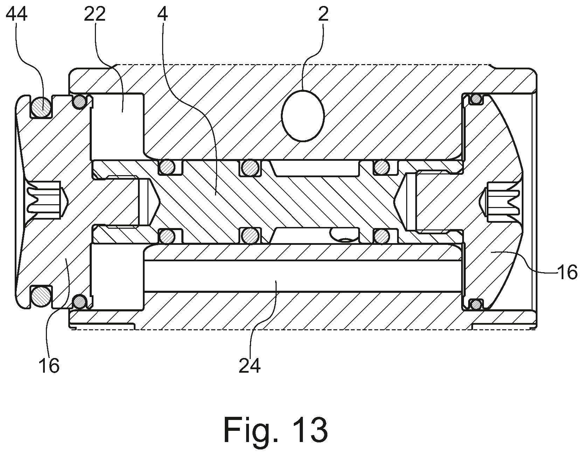

Figur 12 zeigt einen Ausschnitt einer Schnittdarstellung durch ein Schaltventil gemäß einem weiteren Ausführungsbeispiel der vorliegenden Erfindung. Anders als bei anderen gezeigten Ausführungsbeispielen befindet sich der Kanal 24 nicht in der Schaltwelle 4, sondern ist im Ventilgehäuse 2 vorhanden. Er verbindet die beiden Volumina 22, von denen in der gezeigten Position der Schaltwelle 4 nur das rechts dargestellte Volumen 22 bezeichnet ist. Man erkennt zudem an dem links dargestellten Betätigungselement 16 einen Signalring 44, der im gezeigten Ausführungsbeispiel als farblich hervorstechendes Element, beispielsweise in rot oder gelb ausgebildet ist. Er kann als O-Ring beispielsweise aus einem elastischen Material hergestellt sein. In der in Figur 12 gezeigten Position der Schaltwelle 4 ist der Signalring 44 nicht von außen sichtbar. Wird das rechts dargestellte Betätigungselement 16 betätigt und die Schaltwelle 4 in Figur 12 nach links verschoben, wird auch das links dargestellte Betätigungselement 16 nach links verschoben und der Signalring 44 wird von außen sichtbar. Diese Situation ist in Figur 13 dargestellt. Die Schaltwelle 4 befindet sich in der ersten Position, in der das Fußteil 38 und das Unterschenkelteil 36, die in Figur 12 nicht dargestellt sind, relativ zueinander bewegt werden können. In diesem Zustand ist es gegebenenfalls gefährlich, den Prothesenfuß zu belasten, indem beispielsweise der Träger des Fußes auftritt, aufsteht oder geht. Durch den Signalring 44 erhält der Träger ein gut sichtbares Signal, dass die Schaltwelle 4 zuvor in ihre zweite Position, die in Figur 12 gezeigt ist, verschoben werden muss.

### Bezugszeichenliste

- 2: Ventilgehäuse
- 4: Schaltwelle
- 6: Zuflussanschluss
- 8: Abflussanschluss
- 10: Verjüngung
- 12: erstes Dichtelement
- 14: Begrenzungsdichtung
- 16: Betätigungselement
- 18: Ausnehmung
- 20: Dichtung
- 22: Volumen
- 24: Kanal
- 26: Nut
- 28: Ausbuchtung
- 30: Magnet
- 32: Innengewinde
- 34: Außengewinde
- 36: Unterschenkelteil
- 38: Fußteil
- 40: Hydraulikkammer
- 42: Begrenzungselement
- 44: Signalring

## Patentansprüche

1. Prothesenfuß mit einem Fußteil, einem schwenkbar an dem Fußteil (38) angeordneten Unterschenkelteil (36) und einem Hydrauliksystem, wobei das Hydrauliksystem zwei Hydraulikkammern (40) aufweist, die durch eine Hydraulikleitung fluidtechnisch verbunden sind, wobei sich in der Hydraulikleitung ein Schaltventil befindet, das
- ein Ventilgehäuse (2) mit einem Zuflussanschluss (6) und einem Abflussanschluss (8), und
- eine in einem Wellenraum des Ventilgehäuses (2) bewegbare Schaltwelle (4) aufweist,
∘ die in eine erste Position, in der der Zuflussanschluss (6) mit dem Abflussanschluss (8) fluidtechnisch verbunden ist, und
∘ in eine zweite Position bringbar ist, in der der Zuflussanschluss (6) von dem Abflussanschluss (8) fluidtechnisch getrennt ist,
wobei die Einmündung des Zuflussanschlusses und/oder des Abflussanschlusses eine Ausdrehung, Rundung oder Senkung aufweist.

2. Prothesenfuß nach Anspruch 1, **dadurch gekennzeichnet, dass** an wenigstens einem Ende der Schaltwelle (4) ein Betätigungselement (16) angeordnet ist, wobei zwischen dem Betätigungselement (16) und dem Ventilgehäuse (2) eine Dichtung (20) angeordnet ist, die in beiden Positionen der Schaltwelle (4) einen Zwischenraum zwischen dem Betätigungselement (16) und dem Ventilgehäuse (2) abdichtet.

3. Prothesenfuß nach Anspruch 1 oder 2, **dadurch gekennzeichnet, dass** an beiden Enden der Schaltwelle (4) jeweils ein Betätigungselement (16) angeordnet ist, wobei zwischen jedem Betätigungselement (16) und dem Ventilgehäuse (2) eine Dichtung (20) angeordnet ist, die in beiden Positionen der Schaltwelle (4) einen Zwischenraum zwischen dem Betätigungselement (16) und dem Ventilgehäuse (2) abdichtet.

4. Prothesenfuß nach Anspruch 3, **dadurch gekennzeichnet, dass** zwischen dem Ventilgehäuse (2) und jeweils einem Betätigungselement (16) ein Volumen (22) eingeschlossen ist, wobei die beiden Volumina (22) durch einen Kanal (24) fluidtechnisch miteinander verbunden sind, wobei der Kanal (24) vorzugsweise in der Schaltwelle (4) oder in dem Ventilgehäuse (2) vorhanden ist.

5. Prothesenfuß nach einem der vorstehenden Ansprüche, **dadurch gekennzeichnet, dass** die Schaltwelle (4) von der ersten Position in die zweite Position bringbar ist, indem sie entlang ihrer Längsrichtung verschoben wird.

6. Prothesenfuß nach einem der vorstehenden Ansprüche, **dadurch gekennzeichnet, dass** die Schaltwelle (4) von der ersten Position in die zweite Position bringbar ist, indem sie um ihre Längsrichtung gedreht wird.

7. Prothesenfuß nach Anspruch 6, **dadurch gekennzeichnet, dass** die Dichtung (12) wenigstens einen Dichtring aufweist, der so auf der Schaltwelle (4) angeordnet ist, dass die durch den Dichtring definierte Ebene einen Winkel von wenigstens 20°, vorzugsweise wenigstens 30°, besonders bevorzugt wenigstens 40° und höchstens 70°, bevorzugt höchstens 60°, besonders bevorzugt 50° einschließt.

8. Prothesenfuß nach einem der vorstehenden Ansprüche, **dadurch gekennzeichnet, dass** die Schaltwelle (4) um ihre Längsachse drehbar ist, indem ein Druck entlang der Längsrichtung der Schaltwelle (4) auf ein Betätigungselement (16) ausgeübt wird.

9. Prothesenfuß nach Anspruch 8, **dadurch gekennzeichnet, dass** beide Betätigungselemente (16) sich aufeinander zu bewegen, wenn der Druck auf eines der Betätigungselemente (16) ausgeübt wird.

10. Prothesenfuß nach einem der vorstehenden Ansprüche, **dadurch gekennzeichnet, dass** an wenigstens einem der Betätigungselemente (16) und/oder an dem Ventilgehäuse (2) wenigstens ein Permanentmagnet (30) angeordnet ist, der mit einem entsprechenden Gegenelement eine Kraft erzeugt, die die Schaltwelle (4) in die erste Position oder die zweite Position vorspannt.

11. Prothesenfuß nach einem der vorstehenden Ansprüche, **dadurch gekennzeichnet, dass** das Betätigungselement (16) verdrehsicher in einer Aufnahme (18) des Ventilgehäuses (2) geführt sind und vorzugsweise eine ovale Außenkontur und/oder einen Führungsvorsprung, besonders bevorzugt einen Führungsstift, aufweist.

12. Prothesenfuß nach einem der vorstehenden Ansprüche, **dadurch gekennzeichnet, dass** das Schaltventil, vorzugsweise die Schaltwelle (4), über einen Rücksteller verfügt, der eingerichtet ist, die Schaltwelle (4) aus der ersten Position in die zweite Position zurückzusetzen, nachdem die Schaltwelle (4) für eine vorbestimmte Dauer in der ersten Position war.

13. Prothesenfuß nach Anspruch 12, **dadurch gekennzeichnet, dass** der Rücksteller eine hydraulische Zeitverzögerung, die vorzugsweise einstellbar ist, aufweist.

14. Prothesenfuß nach einem der vorstehenden Ansprüche, **dadurch gekennzeichnet, dass** der Rücksteller einen mechatronischen Antrieb aufweist.

15. Prothesenfuß nach einem der vorstehenden Ansprüche, **dadurch gekennzeichnet, dass** die Schaltwelle (4) einen Abschnitt mit verringertem Querschnitt, bevorzugte eine Ausnehmung, eine Nut, eine symmetrische oder asymmetrische Ausdrehung zur Mittelachse der Schaltwelle (4) und/oder eine Abflachung, beispielsweise eine einseitige oder beidseitige Abflachung, aufweist, durch den Hydraulikfluid von dem Zuflussanschluss zu dem Abflussanschluss strömen kann, wenn sich die Schaltwelle (12) in der ersten Position befindet.
